(19) Europäisches Patentamt
European Patent Office
Office européen des brevets

(11) **EP 2 393 588 B1**

(12) **EUROPEAN PATENT SPECIFICATION**

(45) Date of publication and mention of the grant of the patent:
**17.04.2013 Bulletin 2013/16**

(21) Application number: **10705263.1**

(22) Date of filing: **04.02.2010**

(51) Int Cl.:
***B01J 19/30*** *(2006.01)*    ***C12M 1/16*** *(2006.01)*

(86) International application number:
**PCT/DK2010/050034**

(87) International publication number:
**WO 2010/088916 (12.08.2010 Gazette 2010/32)**

(54) **CURVED POLYHEDRONS MADE OF POLYMERS**

KRUMME POLYEDER AUS POLYMEREN

CURVED POLYHEDRONS MADE OF POLYMERS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **05.02.2009 DK 200900175**
**18.12.2009 DK 200901353**

(43) Date of publication of application:
**14.12.2011 Bulletin 2011/50**

(73) Proprietor: **Origo Biotech APS**
**4621 Gadstrup (DK)**

(72) Inventors:
• **DÖRGE, Henrik, Carlheim**
**DK-4621 Gadstrup (DK)**

• **PRANOV, Henrik, Jimenez**
**DK-2400 København NV (DK)**
• **MOGENSEN, Lasse, Wesseltoft**
**DK-2750 Ballerup (DK)**

(74) Representative: **Høiberg, Susanne**
**Høiberg A/S**
**St. Kongensgade 59 A**
**1264 Copenhagen K (DK)**

(56) References cited:
**WO-A2-2005/044972     FR-A- 1 405 374**
**US-A1- 2002 058 456**

**Description**

All patent and non-patent references cited in the application, or in the present application, are also hereby incorporated by reference in their entirety.

**Field of invention**

The present invention relates to a polymer article having the shape of a curved polyhedron characterised by only having concave curvature faces and convex curvature edges or by also having concave curvature faces, in which a part of the concave curvature face has been replaced by a flat face, and convex curvature edges. Another aspect of the present invention regards the use of said polymer article as well as a process for the production of such a polymer article.

**Background of invention**

Polymer carriers are often used in catalytical processes, purification technology and in biological processes such as cell culture. The carriers may be exposed to gas and liquid either freely suspended or densely packed inside a container. Obtaining a high surface-volume ratio of these carriers as well as a high carrier surface - liquid exposure is often desirable.

WO 2005/044972 discloses a three-dimensional carrier for culturing microbiological material. The carrier is in its most generalised form described as a carrier comprising at least two substantially plane, intersecting surfaces with a substantially circular circumference. This can be perceived as a circular polyhedron with cut-outs providing the plane surfaces. While this geometry results in the desired high surface-volume ratio it leaves at the same time sharp edges where the plane surfaces intersect. Sharp edges are well known from the study of Capillary Force Valves within microfluidics (D. Li, 2008, reference 1) to influence the dynamics between the solid, liquid and gas and to give rise to entrapment of gas and liquids which are in contact with carrier because of the capillary effect as well as surface tension between carrier and liquid.

**Summary of invention**

The entrapment of gas bubbles negatively impacts the surface to liquid exposure and thereby the predictability of the biological and catalytical processes. The tendency to entrap gas bubble between polymer surfaces decreases when curvature is present between the surfaces in contrast to sharp edges.

The present invention relates in a first aspect to a polymer article having the shape of a curved polyhedron characterised by only having concave curvature faces and convex curvature edges or by also having concave curvature faces, in which a part of the concave curvature face has been replaced by a flat face, and convex curvature edges. This particular geometry solves the problem of entrapment of gas and liquid which is in contact with the polymer article due to the elimination of sharp edges and thereby reducing the capillary and surface tension effects.

In a particular embodiment the polymer article has been sterilised to a desired Sterility Assurance Level (SAL), such as a SAL of a log of minus 6 of reduction.

In a second aspect the present invention relates to the polymer article of the present invention with a sensitive material or a biological material embedded in or at or coated onto the surface, possibly via an organic molecule capable of covalently linking the biological material.

In a particular embodiment the polymer article with sensitive and/or biological material embedded is sterilised without causing substantial adverse affect to the embedded material.

In a third aspect the present invention relates to the use of said polymer article for use in cell culture.

In a fourth aspect the present invention relates to the use of the polymer article of the present invention for freezing adhering cells from higher organisms e.g. mammals, fish or insects or microorganisms or other biological material comprising the steps of

   a) transferring the polymer article with cells and/or microorganisms and/or biological material to a container
   b) cooling the polymer article of step a) to a temperature below freezing point, such as below 0°C, preferably below minus 78°C.

In a fifth aspect the present invention relates to a container comprising the polymer article of the present invention. The polymer article may be in a non-immobilised or physically immobilised form such as flask, dish, tray, stacked trays, roller bottle, bag, multiwell plate, or elution column

Further aspects and embodiments will be clear from the detailed description of the invention.

**Brief description of figures**

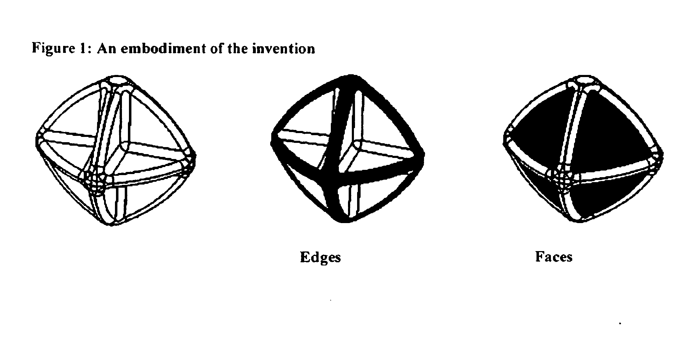

Figure 1 shows a first embodiment of the invention illustrating the determination of edges and faces

Figure 2 shows a second embodiment of the invention illustrating an 8-faced curved polyhedron

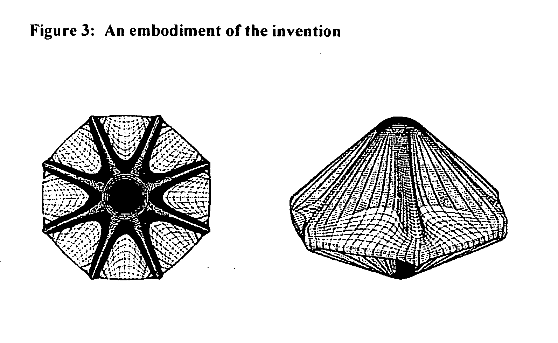

Figure 3 shows a third embodiment of the invention illustrating a 16 faced curved polyhedron

Figure 4 shows a fourth embodiment of the invention illustrating a 2 faced curved polyhedron

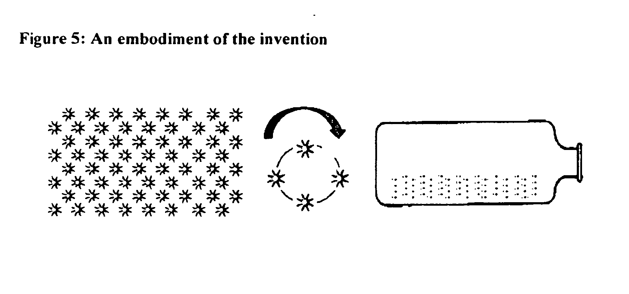

Figure 5 shows a fifth embodiment of the invention illustrating a container comprising elastic deformable grid of polyhedrons

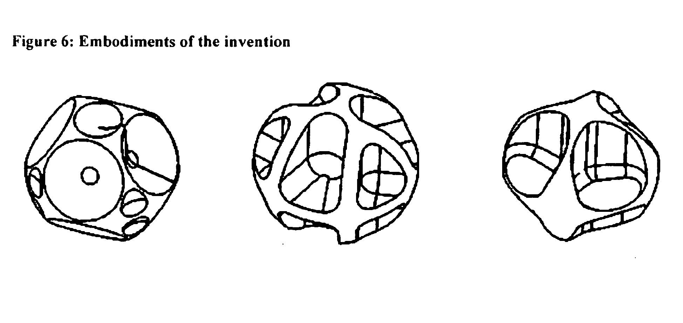

Figure 6 shows a sixth embodiment of the invention

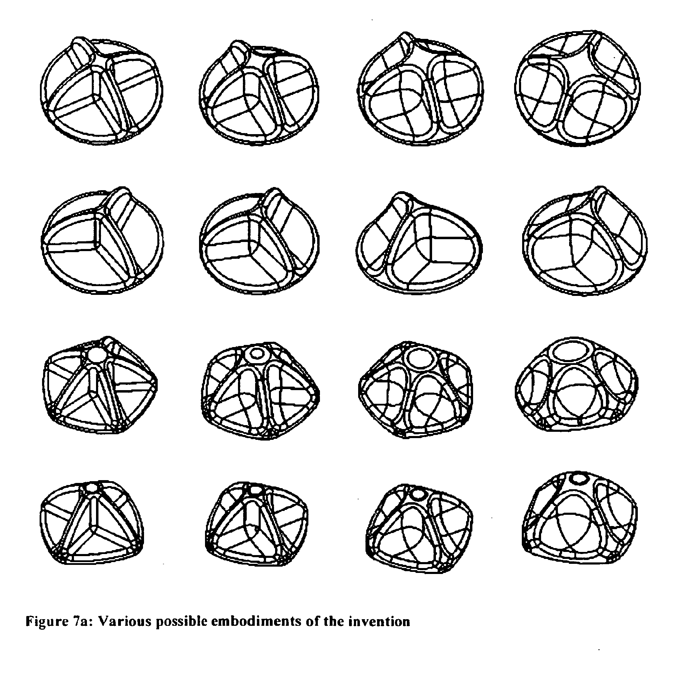

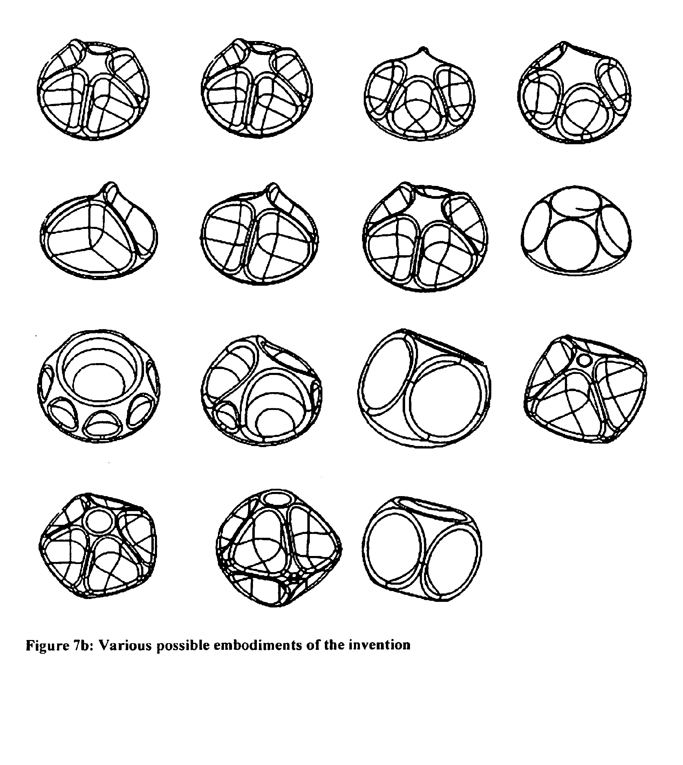

Figure 7a and b: Various embodiments of the invention where the polymer article has a flat base

Figure 8a shows a curved polyhedron, which is a curved cube with 6 concave faces replaced by flat faces

Figure 8b shows a curved polyhedron, which is a sphere with 22 concave faces

Figure 8c shows a curved polyhedron, which is a curved cube with 12 concave faces

Figure 8d shows a curved polyhedron, which is a sphere with 24 concave faces

**Detailed description of the invention**

The present invention relates to a polymer article having the shape of a curved polyhedron characterised by only having concave curvature faces and convex curvature edges or by also having concave curvature faces, in which a part of the concave curvature face has been replaced by a flat face, and convex curvature edges.
In a preferred embodiment the ratio between faces and edges is 1:2 or 2:3.
In another preferred embodiment the polymer article has 2, 4, 5, 6, 8, 10, 12, 14 or 16 faces.
In a preferred embodiment the polymer article is made of a thermoplastic polymer.
In another preferred embodiment the polymer article is sterilised to a Sterility Assurance Level (SAL) of a log of minus 6 of reduction.
In a second aspect the polymer article of the present invention has the shape of a curved polyhedron characterised by only having concave curvature faces and convex curvature edges or by also having concave curvature faces, in which a part of the concave curvature face has been replaced by a flat face, and convex curvature edges, said curved polyhedron comprising of a thermoplastic compound and at least one sensitive material embedded in or at the surface of the thermoplastic compound, characterised in that

a) the at least one sensitive material is sensitive to ionizing irradiation under conditions described in "Sensitivity to ionising irradiation", and
b) the polymeric article may be sterilised by ionizing irradiation while the embedded sensitive material is not substantially adversely affected upon sterilisation to Sterility Assurance Level of a log of minus 6 of reduction.

In a particular embodiment the polymer article disclosed in the above two aspects is produced by injection moulding.
In further embodiments the polymer article disclosed in the above two aspects may be used for cell culture or for handling of frozen biological material.

**Definitions**

Polyhedron is traditionally a three-dimensional geometric shape that is made up of a finite number of flat polygonal faces meeting in pairs along straight edges. A curved polyhedron is a polyhedron with curved faces and edges. This invention provides polyhedrons having only curved surfaces transitioning from concave curvature faces to convex curvature edges, defining the faces as the concave part and the edges as the convex part of the geometry. The concave curvature faces in this invention is closest to the center of the polymer part providing a protected surface, while the convex curvature edges are furthest from the center and have been provided a thickness to retain the required mechanical strength to tolerate the impact of the surroundings without breaking. This invention also provides polyhedrons, in which a part of the concave curvature face has been replaced by a flat face. By introducing this flat face it is easier to bring the microscope in focus viewing the material attached to the flat part of the surface. In principle, any part of the concave curvature face can be replaced by the flat face, but preferably it is the part of the concave curvature face closest to the center of the concave curvature face that has been replaced by the flat face. This replacement does not affect the geometry of the transitioning from the concave curvature faces to convex curvature edges, and the transitioning from the concave curvature face and the flat face is formed as concave edge. No sharp edges can be found in the polymer articles of the present invention.

Outer surface is the part of the surface of the polymer article, which is formed by the convex curvature edges.

Inner surface is the part of the surface, which does not form the outer circumscription of the article, and hence is formed by the concave curvature faces and/or the concave curvature faces, in which a part of the concave curvature face has been replaced by a flat face. Hence, the total area of the outer and the inner surface constitutes the total surface area of the polymer article. Preferably, a large part of the inner surface is protected from being touched or blocked by surfaces or edges of other similar shaped articles due to the constricting geometry of the outer surface. The inner surface is always protected from being touched or blocked by a large flat face of a similar shaped article, which is only able to touch the outer surface of the polymer article.

Protected surface is the part of the inner surface, which is always protected from being touched or blocked by surfaces and edges of other similar shaped articles.. The geometry of the protected surface may take the form of a concave curvature face or the geometry may be a concave curvature face, in which a part of the face closest to the center of the concave face has been replaced by a flat face. Any material attached to such a protected surface is protected from being touched by a similar shaped neighbouring polymer article preventing physical damage to the attached material or detachment of the material from the bead surface.

Effective volume is a measure of the space taken up by article, including the physical volume as well as the space volume protected by the article preventing other articles of similar shape to enter

Sterility Assurance Level (SAL) describes the probability of a single unit being non-sterile after it has been subjected to a sterilisation process.

Sterilisation is the process by which living organisms are removed or killed to the extent that they are no longer detectable in standard culture media in which they had previously proliferated. SAL of a six-log reduction (also termed log of minus 6 reduction or ten to the minus six reduction) is considered acceptable for many medical devices, though overkill cycles may be employed to provide greatest assurance of sterility for critical products such as implantable devices.

Embedded material or surface embedded material means in the context of the present application that the material is embedded in or at the surface of the polymer article. The material may be embedded directly in or at the surface of the polymer or it may be embedded in the polymer via a covalently bound linker covalently bound to the sensitive material. Preferably only a minor part of the material is embedded in or at the surface so that sensitive material maintains its functionality. In a particular embodiment the material is a sensitive material.

Nucleic acids comprise ribonucleic acids, deoxyribonucleic acids and nucleic acid analogs.

Ribonucleic acids comprises RNA, mRNA, pre-mRNA, tRNA, rRNA, anti-sense RNA, guide RNA, microRNA, non-coding RNA, piwi-interacting RNA, small-interferring RNA, small-nucleolar RNA and transfer-messenger RNA.

Deoxyribonucleic acids comprises DNA, cDNA, genomic DNA, multi-copy single-stranded DNA and mitochondrial DNA.

Nucleic acid analogs comprise glycerol nucleic acid, locked nucleic acid, peptide nucleic acid, threose nucleic acid and morpholino.

Lipids are any fat-soluble molecule, such as fat, oils, waxes, cholesterol, sterols, fat-soluble vitamins (vitamins A, D, E and K), monoglycerides, diglycerides and phospolipids. In particular the following categories of lipids are considered: fatty acyls, glycerolipids, glycerophospholipids, sphingolipids, sterol lipids, prenol lipids, saccharolipids and polyketides.

Proteins and peptides are any molecules comprising less than approximately 50 amino acids (peptides) or more than approximately 50 amino acids (proteins) linked covalently together by peptide bonds. The proteins and peptides may be associated with non-protein compounds such as prosthetic groups or they may be glycosylated. Proteins and peptides may be synthesised in vivo by translation of mRNA at the ribosomes by naturally occurring or genetically modified organisms (recombinant expression) or they may be chemically synthesised.

Carbohydrates comprises monosaccharides, disaccharides, oligosaccharides and polysaccharides.

Hormones are chemicals released by cells that affect cells in other parts of the body.

Anchorage dependent cells are cells characterized in that the cells require adhering to a solid substratum for growth, e.g., the solid glass or plastic surface of a culture dish or micro-carrier beads.

Polymer article refers in the following - unless otherwise explicitly specified or evident from the context - to a polymer article having the shape of a curved polyhedron characterized in that it only has concave curvature faces and convex curvature edges.

Biocompatibility is the quality of not having toxic or injurious effects on biological systems.

Biodegradability may be defined by standards well known in the art, such as ASTM D4300, which is a test method designed to be used to determine the susceptibility of polymers to biodegradation.

Sensitive material is in the context of the present invention defined as material where a structural change in macromolecules is caused by heating irradiating the material. Preferred examples of sensitive materials are biological materials. In a particular embodiment sensitive material is material which is sensitive to heat and/or ionising irradiation under conditions specified in the paragraphs "Sensitivity to heat" and "Sensitivity to ionising irradiation" respectively.

Biological material: nucleic acids, peptides, proteins, hormones, lipids carbohydrates, proteins, recombinant proteins, polysaccharides, glycoproteins, proteoglycans, lipoproteins, polypeptides, polynucleotides, oligopeptides, oligonucleotides, hormones, phytohormones, growth factors; materials that comprise a carbohydrate; materials that comprise an antibody or binding fragment of an antibody; materials that comprise a cell adhesion promoting agent or a cell repellent reagent; materials that comprise a component of the extracellular matrix or of blood, interstitial fluid, or other bodily fluids of mammals; materials which are a component of the cell walls of mammals, plants, bacteria, insects or fungi; a mixture of any of any of the aforementioned.

**Polymer article optionally comprising a sensitive material**

The polymer article of the present invention may have any size suitable for the intended use. In particular polymer articles having a diameter of 20 mm or less, such as 15 mm or less, 12 mm or less, 10 mm or less, such as 8 mm or less, 7 mm or less, 6 mm or less, 5mm or less, 4 mm or less, 3 mm or less, 2 mm or less, such as approximately 1 mm.

Micro sized polymer articles may be provided according to the present invention. In particular curved polyhedrons e.g. curved hexagons, with a side length of less than 0.5 mm, such 0.4 mm or less, 0.3 mm or less, 0.2 mm or less, such as 0.15 mm or less, such as 0.125 mm or less, such as 0.10 mm or less, such as 0.05 mm or less.

The polymer article of the present invention is designed in such a way that significant parts of the faces of one polymer article can not be blocked by a neighbouring polymer article making the polymer faces fully exposed to liquid to facilitate and protect the biological processes.

The thickness of the walls is very thin, i.e. substantially less the largest diameter of the polymer article to displace as little liquid as possible while still retaining the required mechanical strength to tolerate the impact of the surroundings without breaking.

The density of the polymer article may be adjusted to fit a specific purpose such as a density below the density of the liquid will allow the articles to float, while a density above the density of the liquid will allow the articles to stay packed even if not fully constrained by a solid wall container. For example the polymer article may be solid or comprise non-solid pockets or various compounds with a higher density than the polymer material, such as magnetic compounds, which may be incorporated into the polymer article affecting density as well as functionality of the article.

In a preferred embodiment the density of the polymer article is within the range of 0.4 $g/cm^3$ to 2.0 $g/cm^3$, such as 0.6 $g/cm^3$ to 1.8 $g/cm^3$, such as 0.8 $g/cm^3$ to 1.4 $g/cm^3$, in particular about 0.9 $g/cm^3$, such as 0.89 $g/cm^3$, or about 1.0 $g/cm^3$, or about 1.05 $g/cm^3$, or about 1.1 $g/cm^3$, or about 1.2 $g/cm^3$, or about 1.3 $g/cm^3$.

The polymer article of the present invention is formed from a heated polymer, in particular a thermoplastic compound. Non-limiting examples of thermoplastic compounds that may be used are acrylonitrile butadiene styrene (ABS), acrylic, celluloid, cellulose acetate, Ethylene-Vinyl Acetate (EVA), Ethylene vinyl alcohol (EVAL), Fluoroplastics, Liquid Crystal Polymer (LCP), polyacetal, polyacrylate, polyacrylonitrile, polyamide, polyamide-imide (PAI), polyaryletherketone, polybutadiene, polybutylene, polybutylene terephthalate, polycaprolactone (PCL), polychlorotrifluoroethylene (PCTFE), polyethylene terephthalate (PET), polycyclohexylene dimethylene terephthalate (PCT), polycarbonate (PC), polyhydroxyalkanoates (PHAs), polyketone (PK), polyester, polyethylene (PE), polyetheretherketone (PEEK), polyetherimide (PEI), polyethersulfone (PES), Polyethylenechlorinates (PEC), polyimide (PI), polylactic acid (PLA), Polymethylpentene (PMP), polyphenylene oxide (PPO), polyphenylene sulfide (PPS), polyphthalamide (PPA), polypropylene (PP), polystyrene (PS), polysulfone (PSU), polyurethane (PU), polyvinyl acetate (PVA), polyvinyl chloride (PVC), polyvinylidene chloride (PVDC) and styrene-acrylonitrile (SAN), or mixes or copolymers thereof.

In a particularly preferred embodiment the polymer article is formed from a biodegradable polymer, such as a biodegradable thermoplast.

Where the polymer article is for use in a biological process, such as cell culture, the polymeric material is biocompatible.

The part of the of the concave curvature face, which may be replaced by a flat face lies in the range of 0 to 90%, such as from 5 to 750%, more preferred from 5 to 55%, even more preferred from 5 to 50% of said concave curvature face.

The polymer article of the present invention may preferably have a total surface area to volume ratio, which lies in the range of 1:1 to 500:1 , preferably in the range of 1:1 to 100:1, most preferred in the range of 1:1 to 5:1.

The total surface area to effective volume ratio of the polymer article preferably lies in the range of 0.50:1 to 5:1, more preferred in the range of 0.8:1 to 3:1, most preferred in the range of 0.9:1 to 2.5:1.

The inner surface area to total surface area ratio of the polymer article lies in the range of 0.5:1 to 0.99:1, more preferred 0.7:1 to 0.97:1 even more preferred in the range of 0.75:1 to 0.96:1.

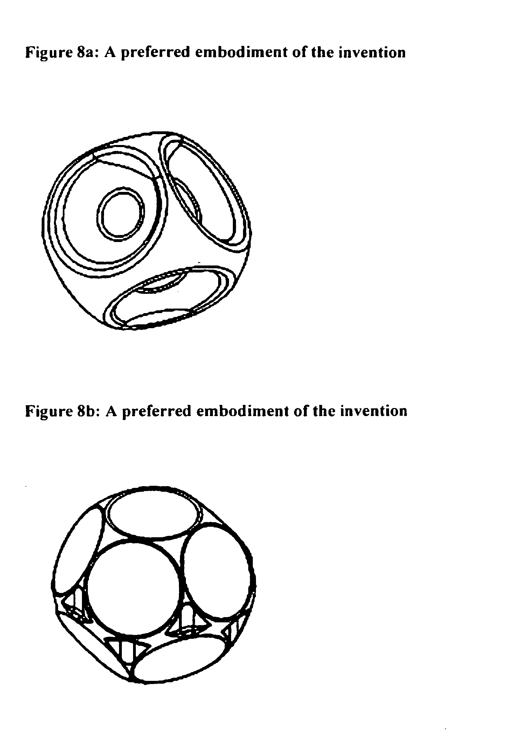

In one preferred embodiment of the invention, the curved polyhedron is formed as a curved cube with convex curvature edges and concave curvature faces, more preferably a curved cube with convex curvature edges and concave curvature faces, in which a part of the concave curvature face has been replaced by a flat face. The part of the concave curvature face, which has been replaced by the flat face is preferably the part, which is closest to the center of the concave curvature face. A preferred embodiment is shown in figure 8a.

In another preferred embodiment of the invention, the curved polyhedron is a sphere with one or more concave grooves. Preferably the number of grooves lies in the range of 12 to 48, more preferred in the range of 16 to 32, even more preferred in the range of 20 to 28. Most preferred the number of grooves is 22. A preferred embodiment is shown in figure 8b.

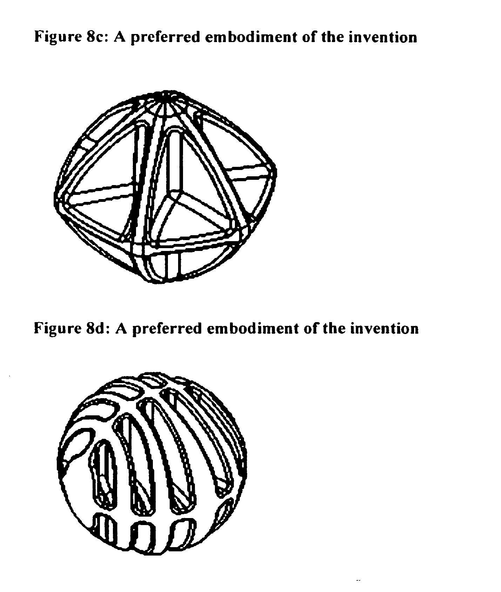

In yet another preferred embodiment of the invention, the curved polyhedron is formed as a curved cube, in which curved tetrahedron-shaped parts of the curved polyhedron have been removed. The number of removed parts preferably lies in the range of 6 to 24 more preferred 8 to 20, even more preferred 10 to 16. Preferably the number of removed parts is 12. A preferred embodiment is shown in figure 8c.

In still another preferred embodiment of the invention, the curved polyhedron is formed as a multifaceted die having concave curvature faces and convex curvature edges. Preferably, the number concave curvature faces lies in the range of 6 to 48, more preferred in the range of 12 to 36. In a most preferred embodiment (as the one shown in figure 8d) the curved polyhedron comprises 24 concave curvature faces.

In one embodiment the polymer article of the present invention is connected to at least one neighbouring polymer article to form a grid of polymer articles. In a particular embodiment the grid is capable of undergoing elastic deformation, i.e. a reversible deformation of the grid resulting in a temporary shape change that is self-reversing after the strain is removed. For example this facilitates the grid being able to enter a small opening in a cell culture flask, and unfold within the flask creating an expanded cell culture area within the flask and allowing easy handling of the flask with the grid being unable to exit through the flask opening.

The polymeric material may be selected such that is has a primarily hydrophobic or primarily hydrophilic surface. In a particular embodiment the surface of the polymer article may be modified to make it more hydrophilic, e.g. by coating or by use of corona or plasma treatment.

The modification may result in a surface exhibiting both hydrophobic and hydrophilic properties. The hydrophilic and hydrophobic areas may blend together randomly or may form pattern, said pattern may be pre-defined.

In an embodiment at least one material is embedded in the polymer article, either in such a way that it is substantially contained by the polymer article or in such a way that it is embedded in or at the surface of the polymer. A process for embedding a material in or at the surface is disclosed in WO 2006/097483. The material may be of biological nature or non-biological nature. Non-biological materials particularly considered are photoluminiscent materials, such as fluorescent or phosphorescent materials, or magnetic materials. The magnetic material may either be a magnet, i.e. material that produces a magnetic field without the application of an external field, or a ferromagnetic or ferrimagnetic material including, but not limited to, iron, nickel, cobalt and some rare earth metals (e.g. gadolinium and dysprosium), alloys comprising the aforementioned materials as well as oxides thereof.

In a particular embodiment a heat sensitive material, possibly a biological material, is embedded in or at the surface of the polymer article. The material optionally embedded is selected from the group materials which are sensitive to heat, i.e. where a structural change in macromolecules takes place upon heating of the material to 100°C for 5 seconds. The heat sensitivity is tested as described in the section "Sensitivity to heat".

In another embodiment a material sensitive to ionising irradiation, possibly a biological material, is embedded in or at the surface of the polymer article. The sensitivity of the material to ionising irradiation is tested as described in the section "Sensitivity to ionising irradiation".

Without being limiting to the present application it is believed that most materials sensitive to ionising irradiation in presence of water are adversely affected by free-radical damage, e.g. lipid peroxidation, denaturation of proteins/peptides and breakdown of polysaccharides to smaller units of carbohydrates. The material sensitive to ionising irradiation under conditions as defined in the section "Sensitivity to ionising irradiation" is, when embedded in or at the surface of the thermoplastic compound of the polymer article of the present application, not substantially adversely affected upon sterilisation. In a preferred embodiment the sterilisation is a SAL of a three-log reduction, such as a SAL of a four-log reduction, such as a SAL of a five-log reduction or a SAL of a six-log reduction. In an even more preferred embodiment overkill cycles have been employed without substantially adversely affecting the embedded material.

In a particular embodiment the sensitive material has been less than 60% degraded or denatured upon sterilisation to SAL of a six-log reduction, such as less than 50% or 40% degraded or denatured upon sterilisation to SAL of a six-log reduction, in a preferred embodiment the sensitive material has been less than 30% degraded or denatured upon sterilisation to SAL of a six-log reduction, such as less than 20% or 10% degraded or denatured upon sterilisation to SAL of a six-log reduction.

The ionising irradiation source applied for the sterilisation of the polymer article is preferably alpha irradiation, beta irradiation, gamma irradiation or X-ray irradiation. The sterilisation comprises an ionising irradition dose of between 1-100 kGy, such as 2-60 kGy, preferably 3-50 kGy, 3-30 kGy or 3-20 kGy. In a preferred embodiment the irradiation dose is in the range of 5-50 kGy, such as 5-40 kGy, 5-30 kGy or 5-25 kGy. In a particularly preferred embodiment the irradiation dose is about 3 kGy, about 4 kGy, about 5 kGy, about 10 kGy, about 15 kGy, about 20 kGy or about 25 kGy.

In a further embodiment the material embedded in or at the surface of the polymer article is sensitive to heat as well as ionising irradiation. Such a sensitive material is in a preferred embodiment selected from the group consisting of peptides, hormones, proteins, nucleic acids, lipids and carbohydrates. Other preferred embodiments are components that increase adhesion of cells to surface (including the polypeptide polylysine), antibodies (mono- or polyclonal) and complement factors.

The present application also concerns a polymer article as described which has been sterilised to a SAL of a three-log reduction, such as to a SAL of a four-log reduction, such as to a SAL of a five-log reduction or even to a SAL of a six-log reduction.

**Process for producing polymer article optionally comprising a sensitive material**

Typical methods used in the mass production of polymer articles are injection moulding or hot embossing.

Injection moulding is performed by heating a suitable polymer until molten, injecting the molten polymer into a mould, allowing the polymer to cool and harden, and removing the moulded article from the mould.

Hot embossing is a process of imprinting structures on a polymer film using a master mold, heat and pressure. The mould is heated above the glass transition temperature of the polymer. Load is applied by pressing the heated mold onto the polymere film using certain embossing pressure. The mold and polymer is cooled below glass transition temperature and the mold is de-embossed. The embossed film may be cut out by ablation, shredding or pouching.

Both injection moulding and hot embossing may be automated and therefore used to produce a rapid succession of articles. The mould used may have means for cooling, in order to increase the speed of hardening of the polymer. Removable inlays with a special surface coating may be incorporated into the mould, and these inlays may bear surface structure and/or texture that are transferred to the polymer article during the injection moulding or embossing process. Alternatively, such structure may be present on the mould.

For general cell culture applications the surface of the polymer article is desirably made hydrophilic to facilitate cell attachment as well as made sterile to prevent contamination.

Typically the surface of the polymer article is modified by corona or plasma treatment to increase surface wettability through electrical discharge.

Sterilisation by irradiation (beta or gamma), steam autoclave, ethylene oxide, chemical disinfectants or dry heat are the typical sterilisation methods used.

The sterile polymer article may in a separate step further be coated using wet chemical process covalently bonding biological molecules to the polymer surface to facilitate specific functionality in relation to interaction with the surroundings being it facilitating cell attachment, molecule capture or separation and/or purification processes.

The many steps involved with typical wet process biological coatings are not optimal in large scale industrial settings from an automated manufacturing point of view resulting in relatively high cost of production as well as an increased product liability as the final biological coated polymer article can not be sterilized after coating due to denaturation of the biological molecules when the wet biological environment on the polymer is exposed to ionizing irradiation or heat.

In advanced biotechnological and medical applications, it is therefore desirable to apply sensitive coatings to defined areas of articles during the manufacturing process in a more dry process and thereby subsequently being able to sterilise the product by ionizing irradiation, preferably alpha, beta or gamma irradiation or X-ray irradiation after the application of the sensitive coating. In a certain embodiment the sensitive material is also sensitive to ionizing irradiation when water is present. The presence of water is defined by the level of water present when passively adsorbing the sensitive material to a polymer surface from an aqueous solution with a subsequent drying for 1 minute in as flow of gaseous, dry nitrogen under conditions that does not adversely affect the sensitive material or the polymer substrate.

It has previously been disclosed WO 2006/097483, by modelling the temperature of the mould in an injection moulding apparatus during injection of the molten polymer, that the molten polymer cools from around 300 °C to 5-10 °C above the mould temperature within 1 micro second of contact between the mould and the molten polymer. The possibility of applying a sensitive material to the surface of a mould covered by a layer of organic molecules comprising a reactive group, or other cooled shaping surface with such a surface coating of a polymer-forming device, and transferring that material intact to the surface of the polymer article, is therefore raised.

Accordingly, in one aspect, the invention provides a method of providing at least one sensitive material on at least part of the surface of a polymer article of the present invention formed from a heated polymer whose temperature is sufficient to adversely affect the at least one sensitive material, wherein:

● a shaping surface is provided having a smooth surface or an at least partly textured surface;
● the at least one sensitive material is applied to at least part of the shaping surface with a layer of organic molecules comprising a reactive group, the shaping surface with said surface coating being at a temperature at which the sensitive material is not adversely affected;
● the heated polymer is brought in contact with the shaping surface with said surface coating;
● the heated polymer is formed by the surface shape of the shaping surface with the said surface coating; and
● the at least one sensitive material is transferred from the shaping surface with said surface coating to the polymer surface; and the temperature of the shaping surface with said surface coating is maintained sufficiently low that the at least one sensitive material is not substantially adversely affected by heat upon or after contact with the heated polymer.

The surface coating of organic molecules comprising a reactive group ensures that the sensitive material may be deposited on the surface coating, and furthermore ensures that the sensitive material may be transferred to the polymer surface. The surface coating shall furthermore ensure that the temperature of the sensitive material is lowered to a temperature that does not adversely affect the sensitive material within a time short enough that an otherwise (long term) adversely affecting (high) temperature does not affect the sensitive material. This may be realised by fulfilling one of two conditions:

1. The thickness of the organic layer comprising a reactive group is sufficiently thin (preferably below 10 nm), as the heat transport properties in the injection moulding apparatus will therefore only be marginally affected, or
2. The heat transfer properties of the organic layer comprising a reactive group are more similar to the heat transfer properties of the mould (high heat capacity, heat conductance and density) than the heat transfer properties of the polymer mould (low heat capacity, heat conductance and low density).

In a preferred embodiment the layer of organic molecules is formed by alkanes, such as halogenated alkanes. In an even more preferred embodiment the layer of organic molecules is a self assembling monolayer.
The layer of organic molecules comprising a reactive group may be bound directly to the shaping surface or bound to an adhesion layer on the shaping surface via the comprised reactive group.
The adhesion layer is selected from the group consisting of metal or semimetal oxides, metal or semimetal nitrides, metal or semimetal carbides or diamond-like carbon.
The reactive group may be selected from the group consisting of halogenated silanes, hydroxylated silanes, or other groups capable of forming covalent bonds to the mould material or adhesion layer.
The at least one sensitive material is maintained at a temperature such that it is not more than 60%, such as not more than 50%, 40% or 30% degraded or denatured by contact with the heated polymer, for example not more than 20% degraded or denatured, or not more than 10% degraded or denatured. Preferably, the at least one sensitive material is not detectably degraded or denatured by the heated polymer. Preferably, the at least one sensitive material has selective binding properties. Preferably, the selective binding affinity of the at least one sensitive material is maintained after contact with the heated polymer.
According to a preferred practice of the invention, the polymer article is formed by injection moulding, said shaping surface covered by a layer of organic molecules comprising a reactive group forming an internal surface of the mould. Alternatively, a different shaping process is used such as compression moulding or calendering. The melting temperature for the polymers typically used in injection moulding or calendering processes is generally between 100°C and 350°C. A suitable temperature for a melt for injection moulding may therefore be up to 350°C, for example 250°C. A suitable temperature for a melt to be formed by calendering may be up to 350°C, for example 250°C.
The temperature of the mould surface must be maintained during polymer injection at a temperature below the denaturation temperature of the sensitive material. This may be achieved by cooling the mould. Preferably, cooling means cooling the mould so that the temperature of the shaping surface is kept at or below 40°C, e.g. by keeping the temperature of the mould below approximately 30°C. This may for instance be a cooling fluid passed through cooling channels in a mould.
The at least one sensitive material is preferably applied to the shaping surface covered by a layer of organic molecules comprising a reactive group by contact with a solution of the sensitive material, for which one may use a liquid dispensing machine or a submersion bath. The areas in which the shaping surface and the solution of the sensitive material are in contact may be an array of smaller homogenous areas or one homogeneous area. The solvent in which the sensitive material is dissolved in is preferably water, or water with additives (e.g. a buffer solution). The contact lasts for a time being preferably lower than one hour and preferably more than one second. More preferably the contact lasts for more than 30 s and less than 10 minutes. After the contact the excess solvent is preferably removed by mechanical means, preferably pressurised gas or a combination of a liquid, preferably water, and pressurised gas. The time after removal of the excess solvent to the time of contact with the polymer is preferably less than 10 minutes, more preferably less than one minute.

The solution contact method and subsequent injection molding process is described in PA 2008 01072. The sensitive material (e.g. collagen) is dissolved in an appropriate solvent (e.g. de-ionised water) in a concentration of e.g. 10 $\mu$g/ml. The solution is pipetted onto the metal inlay in an array of drops. After being in contact for 5 minutes, the drops are removed from the metal inlay by blowing dry, compressed air parallel to the inlay surface. The inlay now contains a corresponding array of surface adsorbed Collagen.

Once the desired combination of materials has been applied to the inlay, the inlay is inserted into the mould. An inlay may be used on each of the two parts of the mold if desired. A molten polymer is then introduced into the mould according to the usual injection moulding technique. The time from the removal of the excess solvent to the introduction of the molten polymer is preferably as short as possible, and in our embodiment less than 30 s.

The molten polymer is allowed to remain in the mould until it has cooled and set. This time may be of the order of a minute or less. After setting, the materials have transferred from the inlay to the surface of the polymer according to the pattern and topography with which the materials were placed on the inlay. The polymer article is removed from the mould, and the materials deposited on its surface may be detected by appropriate methods. If two inlays have been used then the molded polymer article will be coated on both sides. If only one inlay has been used then only one side of the molded polymer article will be coated. It is possible to use only one inlay and mold half of the desired geometry twice with coated material and then assembly the final part before packaging.

The part is packaged in an inert atmosphere, e.g. nitrogen. When packaged it is subject to ionizing irradiation (e.g. gamma or beta or X-ray irradiation) with at dose capable of ensuring a sterility assurance level of at least a four-log reduction, such as a five-log or six-log reduction. The dose depends on the sterility level of the production, but normally doses between 3 and 60 kGy are used. The packaged parts may be subjected to the irradiation at room temperature, or at a lowered temperature, e.g temperature about 0°C or lower, such as about -40°C, -50°C, -60°C, -70°C or -80°C or even lower temperatures such as about -90°C, -100°C, -110°C, -120°C, -140°C, - 160°C, -180°C or -200°C

**Use of sterilised polymeric products**

The polymeric products of the invention may be sterilised without adversely affecting the surface embedded material. They may be used, e.g., in the following applications:

- Implants
- Diagnostics
- Ligand binding processes
- Enzyme immobilisation
- Cell culture
- Personalised medicine
- Cell based bioreactors
- Enzyme based bioreactors
- Lab on chip systems
- Drug screening
- Biosensors
- Separation processes, e.g. chromatography
- Point of care test in microfluidic systems
- Drug delivery
- Freeze and transfer of adhering cells from higher organisms e.g. mammals, fish or insects or microorganisms or other biological material

Cell culture is the process by which prokaryotic or eukaryotic cells are grown under controlled conditions at an appropriate temperature and gas mixture in a cell incubator. Cells can be grown in suspension or adherent cultures. Anchorage dependent cells (or adherent cells) require a surface, such as tissue culture plastic, which may be coated with extracellular matrix components to increase adhesion properties and provide other signals needed for growth and differentiation. Most cells derived from solid tissues are adherent. Another type of adherent culture is organotypic culture which involves growing cells in a three-dimensional environment as opposed to two-dimensional culture dishes. This 3D culture system is biochemically and physiologically more similar to in vivo tissue, but is technically challenging to maintain because of many factors (e.g. diffusion).

The polymer article of the present invention is useful for cell culture, in particular for the culture and upscaling of anchorage dependent cells, because it has a high surface-to-volume ratio and can easily be used within standard cell culture labware and thereby increasing the cell culture support surface without increasing the outer footprint of the container eliminating space related bottlenecks in laboratories and incubators, while maintaining the predictability of the biological process and it can easily be coated with components to increase desired properties, e.g. adhesion properties. Coating material may either be applied to the surface by standard methods known in the art or it may be embedded in or at the surface of the by the method disclosed in the present application.

When stacked loosely or as a grid in containers for cell culture the density of the polymer article is adjusted to avoid floating, e.g. cell culture in flasks, or to allow floating as could be desirable when used as a packed bed (tightly stacked polymer particles) or when supposed to float loosely in fermentors.

Freeze and transfer of microorganisms

The polymer article may be used for freezing adhering cells from higher organisms e.g. mammals, fish or insects or microorganisms or other biological material by transferring the cells, microorganisms or material to the surface of the polymeric material, which may be coated and/or have material embedded in or at the surface, and subsequently freeze the polymer article with biological material using appropriate freezing media. In most cases it is desirable that the polymeric material is sterilised prior to transferring the cells and microorganisms.

In a subsequent step the cells and microorganism may easily be transferred to a container, such as a Petri dish.

The present invention is further described in the following examples which should not be construed as limiting the scope of the invention.

**Experimental**

Sensitivity to ionising irradiation

A test for sensitivity to ionizing irradiation may in the context of the present application be conducted the following way: the material is passively adsorbed to a polymer surface from an aqueous solution with a subsequent drying for 1 minute in a flow of gaseous, dry nitrogen under conditions that does not adversely affect the sensitive material or the polymer substrate. Subsequently the material is irradiated with a dose of 20 kGy of an ionising irradiation.

The material is considered sensitive to ionising irradiation if it is has been denatured or degraded.

Sensitivity to heat

A test for sensitivity to heat may in the context of the present application be conducted the following way: the material is heated to 100°C for 5 seconds in the presence of water, where the presence of water is defined by the level of water present when passively adsorbing the sensitive material to a polymer surface from an aqueous solution with subsequent drying for 1 minute in a flow of gaseous, dry nitrogen under conditions that do not adversely affect the sensitive material or the polymer substrate. For the material to be considered a sensitive material the structural change in the macromolecule preferably leads to denaturation or degradation of the material, i.e. the functionality of the material is impaired upon heating.

Tests for denaturation or degradation are available in the art; see e.g. reference 2 and 3.

**Examples**

Example 1 General procedure for injection moulding

Injection moulding is performed on an Engel 25 tonnes machine fitted with a water-cooled mould with replaceable inlays. The inlays are supported by a highly heat-conducting backplate and coated by a self assembled monolayer of a fluoro-alkane bond covalently through a reactive silane group to an adhesion layer of silicon oxide with a thickness of 10 nm. After application of the media to be transferred, the inlays are mounted in the mould and the polymer injected into the mould. The water cooling is set to the minimum temperature, yielding a mould temperature of 26 °C before injection of the molten polymer. The mould temperature is monitored via a thermistor in the backplate and increased to approximately 30 °C during injection of the molten polymer. The polymer article is removed from the mould after a cooling time of 60 s. The melt temperature used for polystyrene is 250 °C.

Example 2 Procedure for coating the entire shaping surface with Collagen 1 during injection moulding and subsequent irradiation sterilisation

The shape generating surfaces of the polymer article mold is defined on two metallic inlays of the mould cavity. The metallic inlays, made from nickel coated with first an adhesion layer of silicon oxide and second a self assembled monolayer of a covalently bond fluoro-alkane comprising a halogenated silane group, are removed from the cavity and immersed in a solution of the material. Coating with Collagen 1 by immersion in a 10 $\mu$g/ml solution in water for 5-7 minutes. The surface is dried using compressed air. e.g. nitrogen. The injection molding procedure in example 1 is performed. After removal of the final part the parts are packaged in a nitrogen atmosphere. The part is then sterilised using 20 kGy gamma irradiation.

Example 3 Transfer of arrays of Superfibronectin to PS, basic fibroblast growth factor and a mixture thereof with subsequent irradiation sterilisation

The transfer of superfibronectin (SF) /basic fibroblast growth factor (bFGF) /mixture of SF and bFGF is carried out according to the general procedure using inlays coated by use of a solution of 10 micro-g/ml SF in water, 10 micro-g/ml bFGF in water or 10 micro-g/ml SF + 10 micro-g/ml bFGF in water. An array of drops is brought in contact with the inlay. Injection moulding using the inlay with adsorbed SF/bFGF/mixture is carried out according to the general procedure, with the replica being removed from the mould after a cooling time of 60 s. After the part is removed from the mold the part is packed in a nitrogen atmosphere, and irradiated by 25-50 kGy of gamma or beta irradiation. Afterwards human stem cells are cultured on the surface showing good adhesion and no toxic effects.

Example 4 Use of 8-faced curved polyhedrons for cell culture

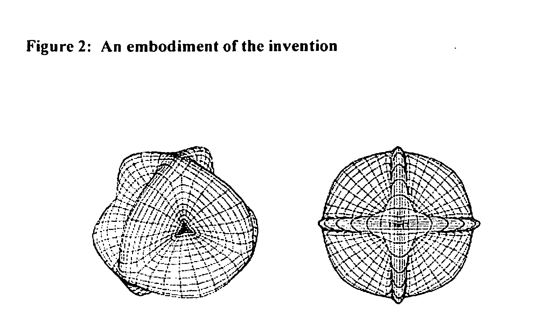

The following example describes one embodiment of use of 8-faced curved polyhedrons for cell culture, e.g. the polyhedron depicted in figure 2.
The polyhedrons have the following characteristica: curved polyhedron with a diameter around 5-7 mm and a density in the range of 1.05 g/cm$^3$ to 1.16 g/cm$^3$.
The curved polyhedrons are presterilized, solid particles. They are further non-absorbing and non-swelling in aqueous media and biocompatible.
The use of the polyhedrons for cell culture may comprise the following steps:

1. Determine the desired surface area/volume ratio (cm$^2$/ml).
2. Weigh out the required amount of Origo Beads under sterile conditions.
3. Determine the number of cells required by multiplying the plating density by the surface area. An inoculum of 1-2 x 10$^4$ cells/cm$^2$ is appropriate for most cell lines, or use an inoculum identical to that used in flasks.
4. Place culture vessels in a laminar flow hood along with the culture medium components which have been pre-warmed to 37°C. Dilute cells into growth medium.
5. Add Origo beads and growth medium with cells to the culture vessel.
6. Incubate cells in a 37°C incubator with 5% $CO_2$ for four days.
If a Roller bottle is used, place this in the Automated Roller Bottle apparatus.
A confluent monolayer of cells should be formed.
7. Evaluate the monolayer when dry. Cells should be at least 75% confluent with good morphology
8. The polymer article with adhering cells may now be

-    transferred to a cryovial with appropriate crymedia and placed in freezer, or
-    washed twice with 1xPBS before trypsination and hereafter go through the consecutive steps 9-12

9. Add trypsin to disaggregate the cells. Incubate culture vessels at 25°C or 37°C and monitor cell detachment under the microscope. Detachment time will vary.
10. After cells detach, add medium to stop trypsinization and to disperse the cells
11. Transfer cells to a sterile conical tube and place on ice.
12. The cells may now be seeded into new cell culture vessel with Origo beads with increased surface area for upscaling of cells.

Example 5 Use of Origo Microcarrier

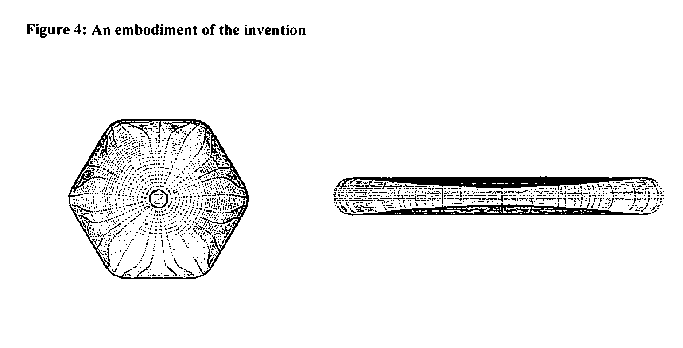

The following example describes one embodiment of use of curved polyhedrons for cell culture, e.g. the polyhedron depicted in figure 4.

The polyhedrons have the following characteristica: curved polyhedron with a side length of approximately 0.125 mm and a thickness of approximately less than 0.025 mm (thinner at the center). The density is in the range of 1.05 g/cm$^3$ to 1.16 g/cm$^3$.

The geometry gives rise to a very high surface-to-volume ratio of approximately 760 cm$^2$/cm$^3$, and therefore also a low medium displacement even at high carrier density (0.13% per cm$^2$/ml). The geometry allows the carrier to float in the liquid media with a minimum of agitation and further prevents neighbouring carriers in sticking to each other by physical interaction

The curved polyhedrons are presterilized, solid particles. They are further non-absorbing and non-swelling in aqueous media and biocompatible.

1. Determine the desired surface area/volume ratio (cm$^2$/ml). The highest successfully used carrier density is 50 cm$^2$/ml. However, a carrier density in the range of 6-15 cm2/ml for initial testing is recommended, especially if the culture vessel has no control of pH, pO2, etc. or medium perfusion.

2. Multiply the surface area/volume ratio by the volume of the culture vessel (working volume) to determine the amount of microcarriers required,e.g.

$$\frac{\text{Vessel Volume (ml) x Area/Volume ratio (cm}^2\text{/ml)}}{760\ (\text{cm}^2\ /\ \text{g})}$$

3. Weigh out the required amount of Origo Microcarriers under sterile conditions e.g.under a laminar air flow apparatus.

4. Add growth medium to the culture vessel, followed by the carriers and finally inoculate the cells. An inoculum of 1-2 x 10$^4$ cells/cm$^2$ is appropriate for most cell lines, or an inoculum identical to that normally used in flasks for the specific cell line may be used.

5. Optimise the stirring/agitation speed, i.e. adjust the stirring speed to the lowest speed which keeps the carriers in uniform suspension. The stirring speed is dependent on the reactor as well as on impeller geometry. The marine type impeller is recommended. It is important for the attachment of the cells that the shear force is as low as possible. The attachment of the cells should occur with continuous stirring to avoid formation of aggregates.

6. When the cells have attached and start proliferating (after 24 hours), it may be necessary to increase the stirring speed slightly to maintain the carriers in suspension. The adhering cell layer (on both sides) increases the total thickness of the carrier considerably, i.e. the total mass of the carrier is increased without increasing the 'floating area'.

**References**

(1) Daniel Irimia in D.Li (Ed). Encyclopedia of Microfluidcis and Nanofluidics 2008; XXXIII,192-196. Capillary Force Valves. ISBN: 978-0-387-32468-5

(2) Gutteridge JM: Int. J Biochem. 1982; 14(7): 649-53: Free-radical damage to lipids, amino acids, carbohydrates and nucleic acids determined by thiobarbituric acid reactivity.

(3) Rael, L. T. et al: Journal of Biochemistry and Molecular Biology, 2004; 37(6): 749-752: Lipid Peroxidation and the Thiobarbaturic Acid Assay: Standardization of the assay when using saturated and unsaturated fatty acids.

**Claims**

1. A polymer article for use in cell culture having the shape of a curved polyhedron **characterised by** having only concave curvature faces and convex curvature edges or by also having concave curvature faces, in which a part of the concave curvature face has been replaced by a flat face, and convex curvature edges, and also by not having sharp edges.

**2.** A polymer article according to claim 1, wherein all faces are concave curvature faces.

**3.** A polymer article according to claim 1, wherein each one of the faces is a concave curvature face, in which a part of the concave curvature face has been replaced by a flat face.

**4.** A polymer article according to claim 1, wherein at least one face is a concave curvature face and wherein at least one face is a concave curvature face, in which a part of the concave curvature face has been replaced by a flat face.

**5.** A polymer article according to any of claims 1-4, wherein the curved polyhedron is formed as a multifaceted die having concave curvature faces and convex curvature edges.

**6.** The polymer article according to any of claims 1-5, wherein:

● the part of the concave curvature face replaced by a flat face lies in the range of 0 to 90% of the concave curvature face; or
● the total surface area to volume ratio lies in the range of 500:1 to 1:1; or
● the total surface area to effective volume ratio lies in the range of 0.5:1 to 5:1.

**7.** The polymer article according to any of claims 1 to 6 which is coated with a biological material or an organic molecule capable of capturing the biological material.

**8.** The polymer article according to any of claims 1 to 7 comprising of a thermoplastic compound and at least one sensitive material embedded in or at the surface of the thermoplastic compound, **characterised in that**

a) the at least one sensitive material is sensitive to ionizing irradiation under conditions described in "Sensitivity to ionising irradiation", and
b) the polymeric article may be sterilised by ionizing irradiation while the embedded sensitive material is not substantially adversely affected upon sterilisation to Sterility Assurance Level of a log of minus 6 of reduction.

**9.** The polymer article according to any of claims 1 to 8 **characterised in that** a photoluminiscent is embedded in or at the surface of the polymer article.

**10.** The polymer article according to any of claims 1 to 9 **characterised in that** it comprises a ferromagnetic or ferrimagnetic material or a material that produces a magnetic field.

**11.** The polymer article according to any of claims 1 to 10 made of a thermo plastic material selected from the group consisting of acrylonitrile butadiene styrene (ABS), acrylic, celluloid, cellulose acetate, Ethylene-Vinyl Acetate (EVA), Ethylene vinyl alcohol (EVAL), Fluoroplastics, Liquid Crystal Polymer (LCP), polyacetal, polyacrylate, polyacrylonitrile, polyamide, polyamide-imide (PAI), polyaryletherketone, polybutadiene, polybutylene, polybutylene terephthalate, polycaprolactone (PCL), polychlorotrifluoroethylene (PCTFE), polyethylene terephthalate (PET), polycyclohexylene dimethylene terephthalate (PCT), polycarbonate (PC), polyhydroxyalkanoates (PHAs), polyketone (PK), polyester, polyethylene (PE), polyetheretherketone (PEEK), polyetherimide (PEI), polyethersulfone (PES), Polyethylenechlorinates (PEC), polyimide (PI), polylactic acid (PLA), Polymethylpentene (PMP), polyphenylene oxide (PPO), polyphenylene sulfide (PPS), polyphthalamide (PPA), polypropylene (PP), polystyrene (PS), polysulfone (PSU), polyurethane (PU), polyvinyl acetate (PVA), polyvinyl chloride (PVC), polyvinylidene chloride (PVDC), styrene-acrylonitrile (SAN), or mixes or copolymers thereof.

**12.** A grid of polymer articles formed by connecting a polymer article according to any of claims 1 to 11 to at least one neighbouring polymer article according to any of claims 1 to 11.

**13.** Use of the polymer article of any of claims 1 to 12 for culturing of microbiological material wherein said microbiological material is adsorbed to the surface of the polymer article.

**14.** The use according to claim 13 wherein the culturing of microbiological material is cell culturing.

**15.** Use of the polymer article of any of claims 1-12 for freezing adhering cells from higher organisms e.g. mammals, fish or insects or microorganisms or other biological material comprising the steps of

a) transfering the polymer article with cells and/or microorganisms and/or biological material to a container
b) cooling the polymer article of step a) to a temperature below freezing point, such as below 0°C, preferably below minus 78°C.

**Patentansprüche**

1. Polymerartikel zur Verwendung bei der Zellkultur, der die Form eines gekrümmten Polyeders aufweist, **dadurch gekennzeichnet, daß** er nur konkave Krümmungsflächen und konvexe Krümmungskanten aufweist oder auch konkave Krümmungsflächen, bei denen ein Teil der konkaven Krümmungsfläche durch eine ebene Fläche ersetzt worden ist, und konvexe Krümmungskanten aufweist, und daß er keine scharfen Kanten aufweist.

2. Polymerartikel nach Anspruch 1, wobei alle Flächen konkave Krümmungsflächen sind.

3. Polymerartikel nach Anspruch 1, wobei jede der Flächen eine konkave Krümmungsfläche ist, bei der ein Teil der konkaven Krümmungsfläche durch eine ebene Fläche ersetzt worden ist.

4. Polymerartikel nach Anspruch 1, wobei wenigstens eine Fläche eine konkave Krümmungsfläche ist und wobei wenigstens eine Fläche eine konkave Krümmungsfläche ist, bei der ein Teil der konkaven Krümmungsfläche durch eine ebene Fläche ersetzt worden ist.

5. Polymerartikel nach einem der Ansprüche 1 bis 4, wobei das gekrümmte Polyeder als ein facettenreicher Würfel ausgebildet ist, der konkave Krümmungsflächen und konvexe Krümmungskanten aufweist.

6. Polymerartikel nach einem der Ansprüche 1 bis 5, wobei

   - der durch eine ebene Fläche ersetzte Teil der konkaven Krümmungsfläche im Bereich von 0 bis 90% der konkaven Krümmungsfläche liegt oder
   - das Verhältnis des Gesamtoberflächenbereichs zum Volumen im Bereich von 500:1 bis 1:1 liegt oder
   - das Verhältnis des Gesamtoberflächenbereichs zum effektiven Volumen im Bereich von 0,5:1 bis 5:1 liegt.

7. Polymerartikel nach einem der Ansprüche 1 bis 6, der mit einem biologischen Material oder einem organischen Molekül beschichtet ist, das fähig ist, das biologische Material aufzubringen.

8. Polymerartikel nach einem der Ansprüche 1 bis 7, umfassend eine thermoplastische Verbindung und wenigstens ein empfindliches Material, das in oder auf der Oberfläche der thermoplastischen Verbindung eingebettet ist, **dadurch gekennzeichnet, daß**

   a) das wenigstens eine empfindliche Material für ionisierende Strahlung unter Bedingungen, wie sie in "Sensitivity to ionising irradiation" beschrieben sind, empfindlich ist, und
   b) der Polymerkartikel durch ionisierende Strahlung sterilisiert werden kann, wobei das eingebettete empfindliche Material bei einer Sterilisation auf einen Sterilisierungsvertrauensgrad von minus 6 Logstufen im wesentlichen nicht nachteilig beeinflußt wird.

9. Polymerartikel nach einem der Ansprüche 1 bis 8, **dadurch gekennzeichnet, daß** ein photolumineszierender Stoff in oder auf der Oberfläche des Polymerartikels eingebettet ist.

10. Polymerartikel nach einem der Ansprüche 1 bis 9, **dadurch gekennzeichnet, daß** er ein ferromagnetisches oder ferrimagnetisches Material oder ein Material, das ein magnetisches Feld erzeugt, umfaßt.

11. Polymerartikel nach einem der Ansprüche 1 bis 10, der aus einem thermoplastischen Material hergestellt ist, das aus der aus Acrylnitrilbutadienstyren (ABS), Acryl, Celluloid, Celluloseacetat, Ethylenvinylacetat (EVA), Ethylenvinylalkohol (EVAL), Fluorkunststoffen, flüssigkristallinem Polymer (LCP), Polyacetal, Polyacrylat, Polyacrylnitril, Polyamid, Polyamidimid (PAI), Polyaryletherketon, Polybutadien, Polybutylen, Polybutylenterephthalat, Polycaprolacton (PCL), Polychlortrifluorethylen (PCTFE), Polyethylenterephthalat (PET), Polycyclohexylendimethylenterephtalat (PCT), Polycarbonat (PC), Polyhydroxyalkanoaten (PHAs), Polyketon (PK), Polyester, Polyethylen (PE), Polyetheretherketon (PEEK), Polyetherimid (PEI), Polyethersulfon (PES), Polyethylenchlorinaten (PEC), Polyimid (PI), Polymilchsäuren (PLA), Polymethylpenten (PM), Polyphenylenoxid (PPO), Polyphenylensulfid (PPS), Polyphthalamid (PPA), Polypropylen (PP), Polystyren (PS), Polysulfon (PSU), Polyurethan (PU), Polyvinylacetat (PVA), Polyvinylchlorid (PV), Polyvinylidenchlorid (PVDC), Styrenacrylnitril (SAN) bestehenden Gruppe, Mischungen oder Copolymeren davon ausgewählt ist.

**12.** Gitter aus Polymerartikeln, das durch Verbinden eines Polymerartikels nach einem der Ansprüche 1 bis 11 mit wenigstens einem benachbarten Polymerartikel nach einem der Ansprüche 1 bis 11 gebildet ist.

**13.** Verwendung des Polymerartikels nach einem der Ansprüche 1 bis 12 zum Kultivieren von mikrobiologischem Material, wobei das mikrobiologische Material an der Oberfläche des Polymerartikels adsorbiert ist.

**14.** Verwendung nach Anspruch 13, wobei das Kultivieren des mikrobiologischen Materials Zellkultur ist.

**15.** Verwendung des Polymerartikels nach einem der Ansprüche 1 bis 12 zum Einfrieren von angehefteten Zellen aus höheren Organismen, wie z.B. Säugetieren, Fischen oder Insekten, oder von Mikroorganismen oder von anderem biologischen Material, umfassend die Schritte des

a) Transferierens des Polymerartikels mit Zellen und/oder Mikroorganismen und/oder biologischem Material in einen Behälter
b) Kühlens des Polymerartikels aus Schritt a) auf eine Temperatur unter dem Gefrierpunkt, wie z.B. unter 0 °C, vorzugsweise unter -78 °C.

## Revendications

**1.** Article polymère destiné à être utilisé dans une culture cellulaire, ledit article ayant la forme d'un polyèdre courbé **caractérisé en ce qu'**il n'a que des faces à courbure concave et des bords à courbure convexe ou **en ce qu'**il a également des faces à courbure concave, dans lesquelles une partie de la face à courbure concave a été remplacée par une face plate, et des bords à courbure convexe, et **en ce qu'**il n'a pas de bord anguleux.

**2.** Article polymère selon la revendication 1, dans lequel toutes les faces sont des faces à courbure concave.

**3.** Article polymère selon la revendication 1, dans lequel chacune des faces est une face à courbure concave, dans laquelle une partie de la face à courbure concave a été remplacée par une face plate.

**4.** Article polymère selon la revendication 1, dans lequel au moins une face est une face à courbure concave et dans lequel au moins une face est une face à courbure concave, dans laquelle une partie de la face à courbure concave a été remplacée par une face plate.

**5.** Article polymère selon l'une quelconque des revendications 1 à 4, dans lequel le polyèdre courbé est formé comme un moule multifacette ayant des faces à courbure concave et des bords à courbure convexe.

**6.** Article polymère selon l'une quelconque des revendications 1 à 5, dans lequel :

la partie de la face à courbure concave remplacée par une face plate se trouve dans la plage de 0 à 90 % de la face à courbure concave ; ou
le rapport total aire de surface/volume se trouve dans la plage de 500/1 à 1/1 ; ou
le rapport total aire de surface/volume effectif se trouve dans la plage de 0,5/1 à 5/1.

**7.** Article polymère selon l'une quelconque des revendications 1 à 6, qui est revêtu d'un matériau biologique ou d'une molécule organique capable de capturer le matériau biologique.

**8.** Article polymère selon l'une quelconque des revendications 1 à 7, comprenant un composé thermoplastique et au moins une matière sensible intégrée dans le composé thermoplastique ou à la surface dudit composé thermoplastique, **caractérisé en ce que**

a) la ou les matières sensibles sont sensibles aux rayonnements dans les conditions décrites dans la section « sensibilité aux rayonnements ionisants », et
b) l'article polymère peut être stérilisé par rayonnements ionisants alors que la matière sensible intégrée n'est sensiblement pas affectée de manière négative au niveau d'assurance de stérilité d'un log de réduction de moins 6.

9. Article polymère selon l'une quelconque des revendications 1 à 8, **caractérisé en ce qu'**un matériau photoluminescent est intégré dans l'article polymère ou à la surface dudit article polymère.

10. Article polymère selon l'une quelconque des revendications 1 à 9, **caractérisé en ce qu'**il comprend un matériau ferromagnétique ou ferrimagnétique ou un matériau qui produit un champ magnétique.

11. Article polymère selon l'une quelconque des revendications 1 à 10, formé en un matériau thermoplastique choisi dans le groupe constitué des matériaux suivants : acrylonitrile butadiène styrène (ABS), acrylique, celluloïde, acétate de cellulose, éthylène-acétate de vinyle (EVA), éthylène-alcool vinylique (EVAL), fluoroplastiques, polymère à cristaux liquides (LCP), polyacétal, polyacrylate, polyacrylonitrile, polyamide, polyamide-imide (PAI), polyaryléthercétone, polybutadiène, polybutylène, téréphtalate de polybutylène, polycaprolactone (PCL), polychlorotrifluoroéthylène (PCTFE), téréphtalate de polyéthylène (PET), polycyclohexylène-diméthylène téréphtalate (PCT), polycarbonate (PC), polyhydroxyalcanoates (PHA), polycétone (PK), polyester, polyéthylène (PE), polyétheréthercétones (PEEK), polyétherimide (PEI), polyéthersulfone (PES), polyéthylènes chlorés (PEC), polyimide (PI), acide polylactique (PLA), polyméthylpentène (PMP), oxyde de polyphénylène (PPO), sulfure de polyphénylène (PPS), polyphtalamide (PPA), polypropylène (PP), polystyrène (PS), polysulfone (PSU), polyuréthane (PU), acétate de polyvinyle (PVA), chlorure de polyvinyle (PVC), chlorure de polyvinylidène (PVDC), styrène-acrylonitrile (SAN), ou des mélanges ou des copolymères de ceux-ci.

12. Grille d'articles polymères formée par connexion d'un article polymère selon l'une quelconque des revendications 1 à 11 à au moins un article polymère voisin selon l'une quelconque des revendications 1 à 11.

13. Utilisation de l'article polymère selon l'une quelconque des revendications 1 à 12, pour la culture d'un matériau microbiologique dans laquelle ledit matériau microbiologique est adsorbé sur la surface de l'article polymère.

14. Utilisation selon la revendication 13, dans laquelle la culture du matériau microbiologique est une culture cellulaire.

15. Utilisation de l'article polymère selon l'une quelconque des revendications 1 à 12, pour congeler les cellules adhérentes d'organismes supérieurs, par exemple, de mammifères, de poissons ou d'insectes, ou de microorganismes ou autre matériau biologique, qui comprend les étapes consistant à :

a) transférer l'article polymère avec les cellules et/ou les microorganismes et/ou le matériau biologique à un récipient ;
b) refroidir l'article polymère de l'étape a) à une température inférieure au point de congélation, par exemple inférieure à 0°C, de préférence inférieure à -78°C.

Figure 1: An embodiment of the invention

Edges          Faces

Figure 2: An embodiment of the invention

**Figure 3: An embodiment of the invention**

Figure 4: An embodiment of the invention

Figure 5: An embodiment of the invention

Figure 6: Embodiments of the invention

Figure 7a: Various possible embodiments of the invention

**Figure 7b: Various possible embodiments of the invention**

Figure 8a: A preferred embodiment of the invention

Figure 8b: A preferred embodiment of the invention

Figure 8c: A preferred embodiment of the invention

Figure 8d: A preferred embodiment of the invention

EP 2 393 588 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005044972 A

- WO 2006097483 A

**Non-patent literature cited in the description**

- Encyclopedia of Microfluidcis and Nanofluidics 2008. Capillary Force Valves. 2008, vol. XXXIII, 192-196
- **GUTTERIDGE JM.** Free-radical damage to lipids, amino acids, carbohydrates and nucleic acids determined by thiobarbituric acid reactivity. *Int. J Biochem.,* 1982, vol. 14 (7), 649-53

- **RAEL, L. T. et al.** Lipid Peroxidation and the Thiobarbaturic Acid Assay: Standardization of the assay when using saturated and unsaturated fatty acids. *Journal of Biochemistry and Molecular Biology,* 2004, vol. 37 (6), 749-752

27